# EUROPÄISCHE PATENTSCHRIFT

(11) **EP 3 094 286 B1**
(45) Veröffentlichungstag und Bekanntmachung des Hinweises auf die Patenterteilung: **11.09.2019**
(21) Anmeldenummer: 15701465.5
(22) Anmeldetag: 08.01.2015
(51) Int. Cl.: A61F 2/50, A61F 2/76, A61F 2/66

(54) **KNÖCHELMANSCHETTE**
ANKLE BRACE
MANCHON DE CHEVILLE

(30) Priorität: 17.01.2014 DE 102014000647
(43) Veröffentlichungstag der Anmeldung: 23.11.2016
(73) Patentinhaber: Otto Bock Healthcare Products GmbH, 1070 Wien (AT)
(72) Erfinder: KAMPAS, Philipp, 1020 Wien (AT); SEYR, Martin, 1040 Wien (AT); SCHNEEGANS, Markus, 37434 Rollshausen (DE); ROST, Roger, 37085 Göttinger (DE); LINTNER, Stephan, 5061 Elsbethen (AT); KULESSA, Sebastian, 37120 Bovenden (DE); MITTERER, Martin, 83435 Bad Reichenhall (DE); VAN MAZIJK, Bernhard, 3004 Riederberg (AT)
(74) Vertreter: Stornebel, Kai
(86) Internationale Anmeldenummer: PCT/EP2015/000015
(87) Internationale Veröffentlichungsnummer: WO 2015/106956

(56) Entgegenhaltungen:
- DE-A1- 10 237 267
- DE-A1- 19 507 894
- DE-A1-102010 049 894
- DE-C- 813 191
- US-A1- 2002 077 703
- US-A1- 2013 326 799

## Beschreibung

Die Erfindung betrifft eine Knöchelmanschette für eine Protheseneinrichtung mit einem Prothesenfuß und einem Unterschenkelteil zur Überbrückung eines zwischen dem Prothesenfuß und dem Unterschenkelteil vorhandenen Freiraumes, mit einem Grundkörper aus einem flexiblen Material, der einen Hohlraum zur Aufnahme der Protheseneinrichtung umfasst. Die Knöchelmanschette dient sowohl als Kosmetik als auch als Schutzumhüllung für Prothesenkomponenten.

Protheseneinrichtungen für untere Extremitäten weisen grundsätzlich einen Prothesenfuß und ein Unterschenkelteil auf, die miteinander verbunden sind. Die Prothesenfüße können an dem Unterschenkelteil auf unterschiedliche Arten und Weisen befestigt werden, neben einer starren Befestigung wird zunehmend eine gelenkige Anordnung des Prothesenfußes an dem Unterschenkelteil vorgenommen. Protheseneinrichtungen, insbesondere solche mit einem Prothesenkniegelenk, weisen häufig Dämpfereinrichtungen, Antriebe und Steuerungen auf, die üblicherweise in dem Unterschenkelteil angeordnet sind. Das Unterschenkelteil nimmt somit Funktionen war, die über die reine Verbindung des Prothesenfußes mit einem Prothesenkniegelenk hinausgehen.

Die Prothesenfüße haben sich von einer starren Imitation des Fußes zu komplexen Bauteilen weiterentwickelt, die eine steuerbare Dämpfung und Komponenten zur Umwandlung mechanischer Energie in elektrische Energie aufweisen können. Darüber hinaus ist eine im Knöchelbereich gelenkige Anlenkung möglich.

Um eine kosmetische ansprechende Ausgestaltung der Protheseneinrichtung zu gewährleisten, werden sogenannte Prothesenkosmetiken eingesetzt. Bei der Ausgestaltung eines Unterschenkelteils als Unterschenkelrohr wird ein Kunststoffschaumkörper in die Form eines Unterschenkels zurechtgeschnitten und modelliert und um das Unterschenkelrohr herumgelegt.

Weiterhin ist es aus dem Stand der Technik bekannt, einen strumpfartigen Überzug zu verwenden, um das Aussehen und die Anmutung einer Protheseneinrichtung an die einer natürlichen Gliedmaße anzunähern. Ein solcher strumpfartiger Überzug ist aus der DE 2130168 A1 bekannt. Dieser strumpfartige Überzug vermindert den Verschleiß von Strümpfen aufgrund eines nicht vorhandenen unmittelbaren Kontaktes mit den Prothesenoberflächen.

Die DE 10 2009 051 441 A1 betrifft eine Kunstgliedhülle mit einer Einführöffnung zum Einführen eines Kunstgliedes, bei der an der Hülle ein Gelenkbereich ausgebildet ist, in den sich ein Endbereich, in den ein Prothesenfuß eingeführt werden kann, einstückig anschließt. Im Gelenkbereich ist eine geringere Shore-Härte als im Endbereich vorhanden. Eine solche Kunstgliedhülle muss um das Kunstglied, in diesem Fall ein Prothesenfuß, herumgelegt werden, was relativ aufwendig ist. Darüber hinaus gibt es Prothesenfüße, die eine dem natürlichen Fuß angenäherte Gestalt aufweisen und keine weitere Umhüllung benötigen.

Die US 2002/077703 A1 betrifft eine Dichtung oder Ummantelung eines Prothesenfußes. Ein Prothesenfußeinsatz ist von einer Fußkosmetik umhüllt, die im Bereich des Knöchels endet und einen oberen Rand aufweist, der radial nach innen absteht. Ein Dichtungselement weist eine umlaufende Nut auf, in die der Rand der Fußkosmetik eingreift. An dem oberen Ende der Dichtung ist ein Dichtring angeordnet oder ausgebildet, der an einem Unterschenkelrohr anliegt.

Die DE 10 2010 049 894 A1 betrifft eine Protheseneinrichtung und eine Verkleidung für eine Protheseneinrichtung mit einem Prothesenfuß, der eine Kosmetik aufweist. Zur Überbrückung eines Spaltes ist ein Halter vorgesehen, der die schalenartige Verkleidung an dem Prothesenfuß fixiert. Der Halter weist einen offenen Querschnitt auf.

Die US2013/326799 A1 betrifft eine Bandage zur topischen Anwendung an einem Menschen zur Verbesserung der Leistungsfähigkeit und Verringerung des Verletzungsrisikos. Die Manschette weist eine flexible Hülse mit seitlichen Fuß-Knöchel-Stützen, Achilles-Sehnen-Stützen und/oder einem verlängerbaren Gurt auf. Die Knöchelmanschette soll während körperlicher Aktivitäten getragen werden, um das Verletzungsrisiko zu reduzieren und die Muskeln eines Sportlers zu trainieren und zu stärken.

Die DE 195 07 894 A1 betrifft eine Unterschenkel-Beinprothese mit einem Prothesenfuß, der ein Fußpassteil aufweist. Ein kosmetisches Knöchelformteil umgreift mit seinem oberen Ende das untere Ende eines Unterschenkelschaftes. An dem unteren Ende des Knöchelformteils ist eine ringförmige Dichtlippe zum spritzwasserdichten Anschluss an das Fußpassteil ausgebildet.

Die DE 813 191 C betrifft ein Knöchelgelenk für künstliche Füße, dessen Armzapfen mit dem Fuß und Unterschenkel starr verbunden sind. Das Knöchelgelenk besteht aus einer Schraubenfeder, die an einem Ende mit dem Unterschenkelteil und an dem anderen Ende mit dem Fußteil verbunden ist. Der Zwischenraum zwischen dem Fußteil und dem Unterschenkelteil ist durch eine an dem Fußteil oder Unterschenkelteil befestigte Manschette abgedeckt.

Die DE 102 37 267 A1 betrifft einen kosmetischen Überzug für eine Prothese mit einer im Wesentlichen unelastischen Kunststoffhülse, die in eine Form vorgedehnt ist, die einem menschlichen Bein ähnelt. Die Kunststoffhülse ist zur lockeren Umhüllung einer Fußprothese, einer schaumüberzogenen Stange und eines Prothesenschaftes ausgelegt und aus einem wärmeschrumpfbaren Kunststoff hergestellt, so dass sich die Kunststoffhülse an die Form der Fußprothese, der Stange und des Prothesenschaftes anpasst. Abschnitte des kosmetischen Überzuges können dicker ausgeführt und/oder mit einem eingearbeiteten Gewebe versehen sein.

Aufgabe der vorliegenden Erfindung ist es, eine Knöchelmanschette bereitzustellen, mit der ein Freiraum zwischen einem Prothesenfuß und einem Unterschenkelteil überbrückt werden kann, wobei eine vielfältige Einsetzbarkeit bei unterschiedlichen Kombinationen von Unterschenkelteil und Prothesenfuß gegeben sein sollte.

Erfindungsgemäß wird diese Aufgabe durch eine Knöchelmanschette mit den Merkmalen des Hauptanspruches gelöst. Vorteilhafte Ausgestaltungen und Weiterbildungen der Erfindung sind in den Unteransprüchen, der Beschreibung sowie den Figuren offenbart.

Die Knöchelmanschette für eine Protheseneinrichtung mit einem Prothesenfuß und einem Oberschenkel zur Überbrückung eines zwischen dem Prothesenfuß und dem Oberschenkel vorhandenen Freiraumes, mit einem Grundkörper aus einem flexiblen Material, der einen Hohlraum zur Aufnahme der Protheseneinrichtung umfasst, sieht vor, dass an dem Grundkörper zumindest ein Versteifungselement zur Erhöhung der Eigenstabilität der Knöchelmanschette angeordnet ist. Die Knöchelmanschette dient zur Überbrückung eines Freiraumes oder Überganges von dem oberen Rand oder dem proximalen Ende des Prothesenfußes und einem Unterschenkelteil, beispielsweise einem Gehäuse einer Dämpfer- und Steuereinrichtung oder einer anderen, das Volumen eines natürlichen Unterschenkels nachbildenden und/oder die Funktion eines Unterschenkelrohrs ausübenden Komponente. Dazu weist die Knöchelmanschette einen Grundkörper auf, der aus einem flexiblen Material besteht. Der Grundkörper bildet im angelegten Zustand einen Hohlraum aus, in dem die Protheseneinrichtung, überwiegend ein Teil des Unterschenkelteils, angeordnet ist. Der Grundkörper umgibt somit einen distalen Abschnitt des Unterschenkelteils im Bereich des Überganges zum Prothesenfuß. An dem Grundkörper ist ein Versteifungselement angeordnet, beispielsweise angeklebt, angeschweißt, angespritzt oder angeformt oder auf eine andere Art und Weise befestigt, um einerseits eine Optik zu erzeugen, die dem natürlichen Knöchelbereich oder distalen Unterschenkelbereich entspricht und andererseits eine funktionale Abdeckung der mechanischen Komponenten der Protheseneinrichtung bereitzustellen. Das Versteifungselement ermöglicht es, dass die Knöchelmanschette eine ausreichende Formstabilität beim Bewegen der Protheseneinrichtung aufweist, ohne dass die Beweglichkeit zu sehr eingeschränkt wird. Ebenso ist eine ausreichende Verformbarkeit durch den flexiblen Grundkörper gewährleistet, so dass eine natürliche Anmutung weiterhin gegeben ist. Über das Versteifungselement ist es möglich, die Funktionalität der Knöchelmanschette an die gewünschten Eigenschaften anzupassen, nämlich dass eine ausreichend große Stabilität vorhanden ist, um ein Herunterrutschen oder ein Abrutschen der Manschette zu verhindern, ohne dass eine Flexibilität verloren geht, die benötigt wird, um ein Falten oder Umschlagen der Manschette zu vermeiden, wie es bei einem Einknicken eines starren Materials, etwa dem eines Gummistiefels der Fall wäre. Die Erfindung sieht vor, dass das Versteifungselement als ein Rahmen ausgebildet ist, der den Grundkörper umgibt. Der Rahmen kann umlaufend um den gesamten Umfang des Grundkörpers herum angeordnet sein, so dass eine ausreichende Formstabilität zur Montage der Knöchelmanschette beispielsweise an dem Prothesenfuß oder an dem Unterschenkelteil gegeben ist. Durch den Rahmen wird der Hohlraum, der von dem Grundkörper umgeben wird, definiert, so dass der Grundkörper selbst aus einem biegeschlaffen Material ausgebildet sein kann, das durch das Versteifungselement in der gewünschten Form gehalten wird.

Das Versteifungselement besteht dabei aus einem Material, das einen gegenüber dem Material des Grundkörpers größeren Verformungswiderstand aufweist, so dass gezielt an denjenigen Stellen, an denen das Versteifungselement angeordnet ist, die gewünschte Formstabilität und der gewünschte Verformungswiderstand erzielt bzw. erhöht werden kann. Ebenso ist es möglich, durch das Versteifungselement an besonders empfindlichen Stellen im Übergang zwischen dem Prothesenfuß und dem Unterschenkelteil eine verbesserte mechanische Schutzwirkung bereitzustellen.

Der Grundkörper kann aus einem Textil und/oder einem Schaumstoff hergestellt sein bzw. ein Textil und/oder einen Schaumstoff aufweisen und weitere Materialien umfassen, sofern dies notwendig ist. Textile und Schaumstoffe haben den Vorteil einer einfachen Herstellbarkeit, Verarbeitbarkeit und eines leichten Gewichtes bei ausreichender Flexibilität und Verformbarkeit. Die Textilien können als Gewebe, Gewirke, Gestricke und als Abstandsgewirke ausgebildet sein, als Schaumstoffe sind sowohl offenporige als auch geschlossenporige Schaumstoffe vorgesehen. Der Grundkörper kann eine Beschichtung aufweisen, die die Wasserundurchlässigkeit der Knöchelmanschette erhöht oder herstellt.

Alternativ kann der Grundkörper des Überbrückungselementes auch aus einem Elastomer, beispielsweise aus einem Silikon oder TPE oder einem weichgemachten Thermoplast, beispielsweise einem Weich-PVC hergestellt werden.

Zum formschlüssigen Festlegen der Knöchelmanschette an dem Prothesenfuß können Befestigungselemente an dem Grundkörper oder dem Versteifungselement angeordnet sein, so dass eine Relativbewegung zwischen der Knöchelmanschette und dem Prothesenfuß während der üblichen Benutzung der Protheseneinrichtung nicht erfolgen kann. Dadurch ist es möglich, die Knöchelmanschette bündig oder nahezu bündig mit dem proximalen Abschluss des Prothesenfußes auszubilden bzw. zu orientieren. Die Befestigungselemente zum formschlüssigen Festlegen bewirken zudem, dass sich die Kontur der Knöchelmanschette an die Kontur des Prothesenfußes anpasst, so dass es möglich ist, unterschiedliche Formen von Prothesenfüßen mit ein und derselben Knöchelmanschette zu kombinieren.

In dem Versteifungselement kann zumindest eine Scharniereinrichtung zur Erleichterung einer Flexion um ein Knöchelgelenk angeordnet sein. Diese Scharniereinrichtung erleichtert die Relativbewegung zwischen dem Prothesenfuß und dem Unterschenkelteil und verhindert, dass Geräusche beim Einbeugen durch ein Auffalten der Knöchelmanschette entstehen. Weiterhin werden die Auszugskräfte verringert, die bei einer Flexion um ein Knöchelgelenk, sei es eine Plantarflexion oder eine Dorsalflexion, auftreten. Durch die Reduzierung der Auszugskräfte wird gewährleistet, dass die Knöchelmanschette in der vorgesehenen Position relativ zu dem Prothesenfuß oder dem Unterschenkelteil verbleibt.

Die Knöchelmanschette ist vorteilhafterweise symmetrisch ausgebildet, wobei die Symmetrie vorzugsweise zur Sagittalebene besteht, so dass eine Manschette sowohl für die Anwendung an einer linken als auch an einer rechten Protheseneinrichtung geeignet ist.

Das Versteifungselement kann den distalen Abschluss der Knöchelmanschette bilden, wobei zumindest Teile des Versteifungselementes den distalen Abschluss gestalten. Dadurch ist es möglich, dass insbesondere Befestigungselemente, die naturgemäß eine größere Festigkeit als das biegeschlaffe oder flexible Material des Grundkörpers aufweisen müssen, leichter hergestellt und angeformt werden können. Bei einer separaten Ausgestaltung der Befestigungselemente ist es möglich, durch die Anordnung am distalen Ende der Knöchelmanschette eine einfache Zuordnung der Befestigungselemente sowohl zu der Knöchelmanschette als auch zum Prothesenfuß zu erreichen. Die Befestigungselemente können dabei formschlüssig an einem Formschlusselement an der Knöchelmanschette festgelegt werden, das an dem Versteifungselement ausgebildet oder befestigt ist.

Auf der Innenseite, insbesondere im proximalen Bereich des Grundkörpers kann eine reibungsvermindernde Beschichtung angeordnet sein, ebenso können reibungsvermindernde Elemente auf der Innenseite des Grundkörpers angeordnet sein, beispielsweise angeklebt, angespritzt, angeschweißt oder auf andere Art und Weise an dem Grundkörper befestigt sein. Durch eine reibungsvermindernde Beschichtung ist es möglich, eine Relativbewegung zwischen der Knöchelmanschette und dem Unterschenkelteil zu ermöglichen, ohne dass hohe Auszugskräfte auf die Knöchelmanschette wirken, so dass eine feste Zuordnung zwischen dem distalen Ende der Knöchelmanschette und dem proximalen Ende des Prothesenfußes erhalten bleibt.

Das Versteifungselement kann einen geschlossenen Querschnitt aufweisen, um die Formstabilität weiter zu erhöhen. Die Form ist in der Regel oval oder einem Oval angenähert. Das Versteifungselement kann sich über einen größeren Höhenbereich in Proximal-Distal-Erstreckung erstrecken, wobei Ausschnitte in dem Versteifungselement in anteriorer und/oder posteriorer Orientierung vorgesehen sein können. Durch diese Ausschnitte ist es möglich, eine Verformung des Grundkörpermaterials zu ermöglichen. Dazu ist es vorgesehen, dass das Versteifungselement auf dem Grundkörper aufgebracht ist, so dass die gegebenenfalls vorgesehenen Ausschnitte in dem Versteifungselement von dem Grundkörpermaterial abgedeckt sind. Die Knöchelmanschette ist somit im angelegten Zustand vorzugsweise geschlossen, so dass keine Feuchtigkeit und/oder Schmutz eindringen kann.

Alternativ zu einem geschlossenen Querschnitt des Versteifungselementes ist es vorgesehen, dass der Grundkörper einen offenen Querschnitt aufweist und das Versteifungselement auch oder nur entlang der Öffnung angeordnet ist und den Querschnitt schließt, also die einander gegenüberliegenden Kanten des Zuschnittes des Grundkörpers aufnimmt, fixiert und insgesamt einen geschlossenen Querschnitt für die Knöchelmanschette bereitstellt.

Nachfolgend werden Ausführungsbeispiele der Erfindung sowie nicht erfindungsgemäße Varianten zu Illustrationszwecken anhand der beigefügten Figuren näher erläutert: Es zeigen:
- Figur 1: eine Seitenansicht einer Protheseneinrichtung mit Knöchelmanschette;
- Figur 2: eine Ansicht einer nicht erfindungsgemäßen Knöchelmanschette von schräg hinten;
- Figur 3: eine Einzeldarstellung einer erfindungsgemäßen Knöchelmanschette;
- Figur 4: eine Seitenansicht gemäß Figur 3;
- Figur 5: eine Rückansicht gemäß Figur 3;
- Figur 6: eine Frontalansicht gemäß Figur 3;
- Figur 7: eine Einzelteildarstellung einer nicht erfindungsgemäßen Variante;
- Figur 8: eine Detailansicht der Variante gemäß Figur 7;
- Figur 9: eine Teilschnittdarstellung der Variante gemäß Figur 7 in Schrägdraufsicht; sowie
- Figur 10: eine weitere Variante in Seitenansicht.

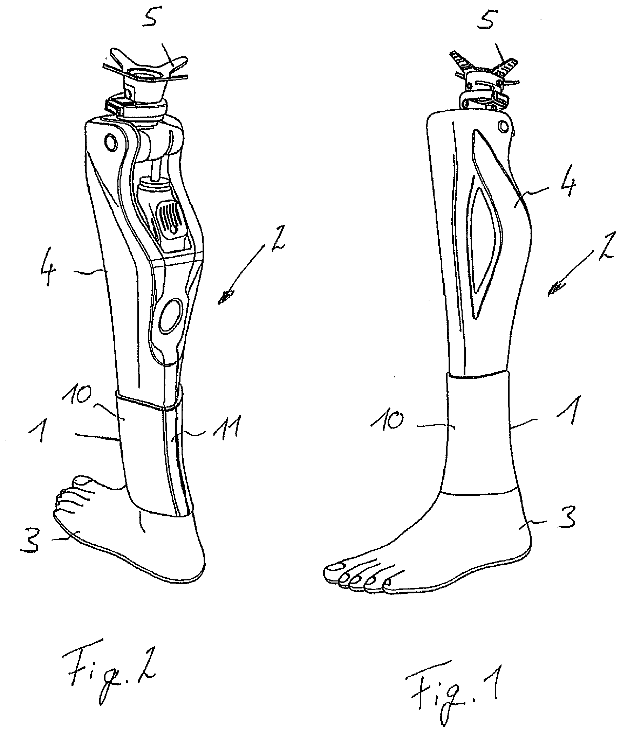

In der Figur 1 ist in einer Gesamtansicht eine Protheseneinrichtung 2 gezeigt, die einen Prothesenfuß 3, ein an dem proximalen Ende des Prothesenfußes 3 befestigten Unterschenkelteil 4 und eine am proximalen Ende des Unterschenkelteils 4 angeordneten Befestigungseinrichtung 5 aufweist. Die Befestigungseinrichtung 5 dient zur Montage der Protheseneinrichtung 2 an einem nicht dargestellten Oberschenkelschaft. Die Befestigungseinrichtung 5 ist gelenkig an dem Unterschenkelteil 4 gelagert und somit Teil eines Prothesenkniegelenkes. Oberhalb des Prothesenfußes 3, also im Anschluss an das proximale Ende des Prothesenfußes 3, ist eine Knöchelmanschette 1 angeordnet, die einen Grundkörper 10 aufweist. Die Knöchelmanschette 1 überdeckt den Übergangsbereich von dem Prothesenfuß 3 zum Unterschenkelteil 4 und erstreckt sich ungefähr über ein Drittel der Länge des Unterschenkelteils 4. Die Knöchelmanschette 1 umgibt den Unterschenkelteil 4 vollständig und ist an dem Prothesenfuß 3 über nicht dargestellte Formschlusselemente reversibel festgelegt.

In der Figur 2 ist die Protheseneinrichtung 2 mit einer nicht erfindungsgemäßen Knöchelmanschette 1 von schräg hinten gezeigt. In dem Unterschenkelteil 4 sind Dämpfereinrichtungen und eine Steuerelektronik sowie das Prothesenkniegelenk zu erkennen. Die Knöchelmanschette 1 selbst weist an der posterioren Seite ein an dem Grundkörper 10 angeordnetes Versteifungselement 11 auf, das an der posterioren Seite der Protheseneinrichtung 2, also im Bereich der Wade angeordnet ist und sich im Wesentlichen in Längsrichtung des Unterschenkelteils 4 erstreckt. Das Versteifungselement 11 dient einerseits zur Gewährleistung der Stabilität der Knöchelmanschette 1 in deren Längserstreckung und verhindert, dass während der Benutzung der Knöchelmanschette 1 diese von dem Unterschenkelteil 4 in Richtung auf den Prothesenfuß 3 herunterrutscht und gegebenenfalls Falten schlägt. Das Versteifungselement 11 dient weiterhin zum Verbinden der einander gegenüberliegenden Enden des Grundkörpers 10, der im dargestellten Ausführungsbeispiel nicht als kreisförmiger oder ovaler Materialzuschnitt mit einem geschlossenen Querschnitt, sondern als ein Flachzuschnitt mit einem offenen Querschnitt ausgebildet ist, wobei die einander gegenüberliegenden Kanten des Zuschnittes über das Versteifungselement 11 miteinander verbunden werden, so dass ein Hohlraum zur Aufnahme der Protheseneinrichtung 2, insbesondere des unteren oder distalen Teils des Unterschenkelteils 4 bereitgestellt wird.

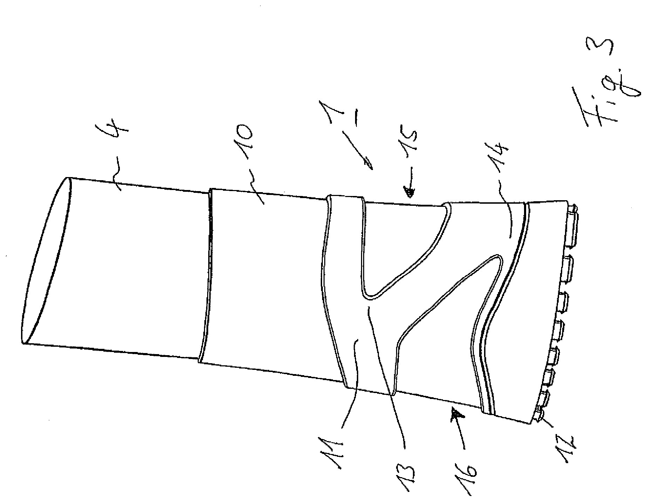

In der Figur 3 ist eine erfindungsgemäße Knöchelmanschette 1 gezeigt, die an einem Unterschenkelteil 4 angeordnet ist. Der Prothesenfuß ist in der Figur 3 nicht dargestellt. Die Knöchelmanschette 1 sieht einen Grundkörper 10 aus einem flexiblen Material, insbesondere einem Textil, einem Schaumstoff oder einer Kombination mehrerer flexibler Werkstoffe vor. Der Grundkörper 10 ist im dargestellten Ausführungsbeispiel schlauchartig ausgebildet und weist einen geschlossenen Querschnitt auf. Auf der Außenseite des Grundkörpers 10 ist ein Versteifungselement 11 in Gestalt eines Rahmens angeordnet, der unter anderem einen umlaufenden, distalen Abschluss der Knöchelmanschette 1 ausbildet. An dem distalen Ende des Versteifungselementes 11 sind Befestigungselemente zur formschlüssigen Festlegung an dem Prothesenfuß ausgeformt. Neben der dargestellten einstückigen Variante mit angeformten Befestigungselementen 12 ist es möglich, an dem Versteifungselement 11 separate Befestigungselemente festzulegen, um diese dann mit Formschlusselementen an dem Prothesenfuß in Eingriff zu bringen und dort eine formschlüssige Festlegung der Knöchelmanschette 1 an dem Prothesenfuß zu bewirken.

An dem distalen Ende des Grundkörpers 10 ist das Versteifungselement 11 als umlaufender geschlossener Ring ausgebildet, von dem sich von dem posterioren Ende, das in der Figur 3 rechts ausgebildet ist, ein schräg nach oben verlaufender Rahmenabschnitt verläuft. Der Übergang von dem unteren, ringförmigen Abschluss zu dem nach oben zeigenden Rahmenabschnitt ist als eine Scharniereinrichtung 14 ausgebildet, so dass eine federnde Bewegung des anterioren, oder vorderen Bereiches des Versteifungselementes 11 nach unten oder in distaler Richtung erfolgen kann. Dazu ist in dem Versteifungselement 11 eine anteriore Ausnehmung 16 vorgesehen, die von dem Material des Grundkörpers 10 ausgefüllt ist.

Von dem sich schräg nach vorn erstreckenden Rahmenabschnitt des Versteifungselementes 11 erstreckt sich leicht nach oben geneigt ein oberer Teil schräg nach hinten und nach oben, also zu dem proximalen Ende der Knöchelmanschette 1 hin, und bildet so einen Bügel aus, der sich um den posterioren Teil des Grundkörpers 10 oberhalb des posterioren Scharniers 14 herum erstreckt. An dem Übergang von dem schräg nach vorne weisenden Rahmenabschnitt zu dem schräg nach hinten weisenden Rahmenabschnitt ist ein zweites Scharnierelement oder eine zweite Scharniereinrichtung 13 angeordnet und ausgebildet, so dass der sich schräg nach hinten erstreckende Rahmenabschnitt nach unten verlagert werden kann. Unterhalb des oberen Rahmenabschnittes ist eine posteriore Ausnehmung 15 ausgebildet, die ebenfalls von dem Material des Grundkörpers 10 verschlossen ist. Wird eine Plantarflexion des Prothesenfußes 3 vorgenommen, ergibt sich analog zu einer natürlichen Fußbewegung eine Verlagerung des oberen, nach hinten weisenden Rahmenabschnittes um die Scharniereinrichtung 13 und eine flexible Verformung des Materials des Grundkörpers 10 innerhalb der Ausnehmung 15 durch eine Stauchung, während das flexible Material des Grundkörpers 10 im Bereich der anterioren Ausnehmung 16 gestreckt wird und der sich nach vorne erstreckende Rahmenteil nach oben verlagert wird. Bei einer Dorsalflexion erfolgt eine Stauchung im Bereich der anterioren Ausnehmung 16 und eine Streckung im Bereich der posterioren Ausnehmung 15. Neben einer einteiligen Ausgestaltung des Versteifungselementes 11 ist es vorgesehen, dass es mehrteilig oder modular aufgebaut ist, so dass beispielsweise der untere, ringförmige Abschluss mit den oberen Rahmenabschnitten verbunden ist, ggf. gelenkig über ein Scharnier oder mehrere Scharniere verbunden.

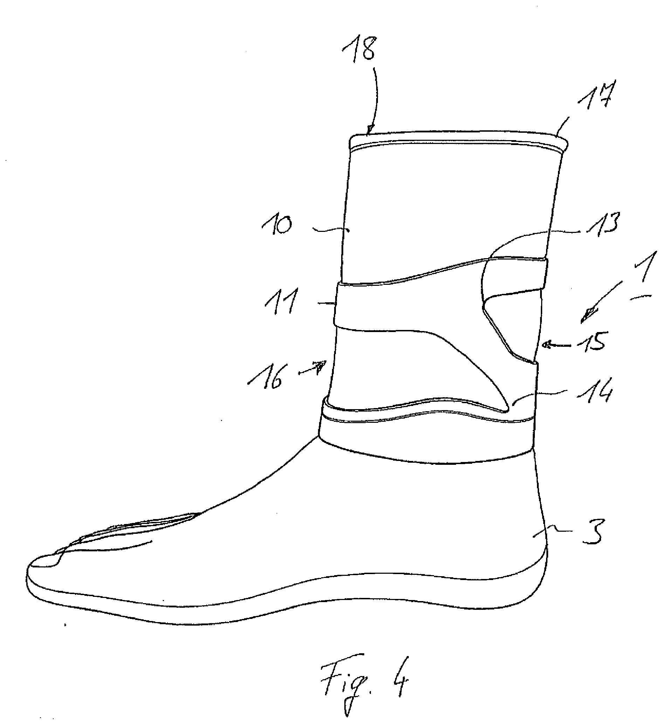

Figur 4 zeigt die Ausführungsform gemäß Figur 3 in einem an dem Prothesenfuß 3 montierten Zustand in Seitenansicht. Die Befestigungselemente 12 sind in nicht dargestellten Ausnehmungen im oberen Rand des Prothesenfußabschlusses eingeführt und ermöglichen es, eine bündige Montage der Knöchelmanschette 1 an dem Prothesenfuß 3 durchzuführen. Neben einer sicheren Festlegung gegen eine Auszugsbewegung, also von dem Prothesenfuß 3 weg, erfolgt über die Befestigungselemente 12 auch eine Anpassung der Knöchelmanschette 1 an die Kontur des jeweiligen Prothesenfußes 3, da neben dem Grundkörper 10 auch das Versteifungselement eine Flexibilität und Elastizität aufweist, so dass unterschiedliche Prothesenfüße 3 mit nur einer Knöchelmanschette 1 versorgt werden können. In der Figur 4 ist zu erkennen, dass das Versteifungselement 11 den Grundkörper 10 in der gewünschten Form hält, so dass der Grundkörper 10 einen Hohlraum umschließt, der über den proximalen Rand des Versteifungselementes 11 hinausragt. Auf der Innenseite 18 am proximalen Rand 17 des Grundkörpers 10 kann eine reibungsvermindernde Beschichtung 18 angeordnet sein, um eine Relativbewegung zwischen dem nicht dargestellten Unterschenkelteil 4 und dem Material des Grundkörpers 10 zu erleichtern.

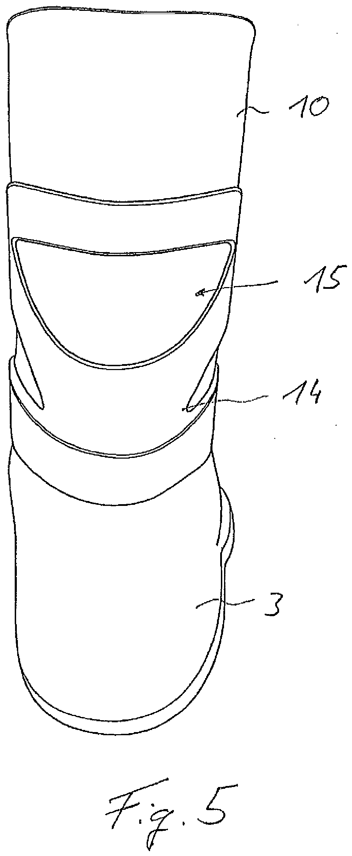

Figur 5 zeigt die Ausführungsform gemäß der Figur 4 in Rückansicht, es ist der Prothesenfuß 3 mit dem im Umfang bündig abschließenden, umlaufenden Versteifungselement 11 zu erkennen. Im posterioren Bereich, also im Bereich der Achillesferse, ist oberhalb des distalen Ringes des Versteifungselementes 11 die Scharniereinrichtung 14 ausgebildet, die durch die Ausnehmung 16 ausgebildet wird. Die Scharniereinrichtung 14 ermöglicht es, dass der sich nach oben, also zum proximalen Ende der Knöchelmanschette 10 erstreckende Teile oder Abschnitt des Versteifungselementes 11 leicht eingebeugt werden kann, bei dem posterioren Scharnier 14 bedeutet dies, dass die sich nach oben anschließenden Abschnitte des Versteifungselementes 11 bei einer Dorsalflexion nach vorne und unten verlagern können, also in Richtung auf den Prothesenfuß. Bei einer Plantarflexion übernimmt das obere bzw. anteriore Scharnier 13 diese Funktion und ermöglicht es dem posterioren, proximalen Abschnitt des Versteifungselementes 11 eine Verlagerung in Distalrichtung, so dass die posteriore Ausnehmung 15 verkleinert wird und das Material des Grundkörpers 10 komprimiert bzw. gestaucht wird.

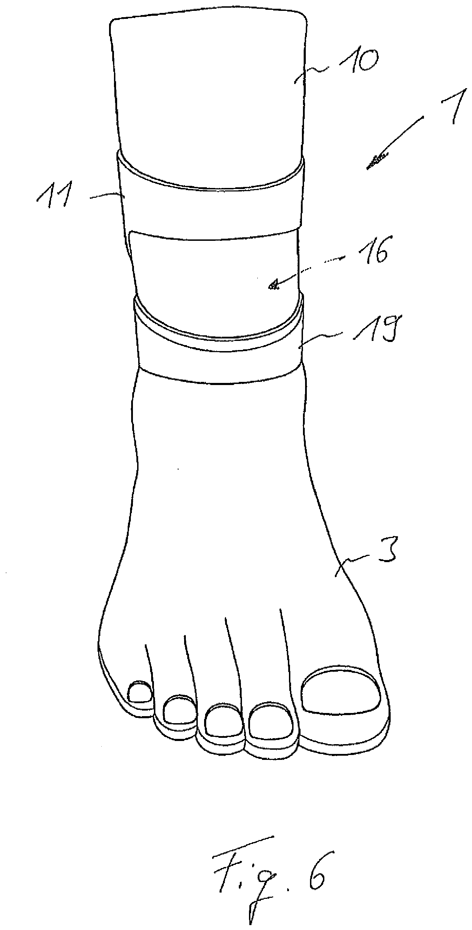

Figur 6 zeigt die Ausführungsform gemäß der Figuren 4 und 5 in einer Frontalansicht. Es ist zu erkennen, dass das Versteifungselement 11 im anterioren Bereich eine Ausnehmung 16 aufweist, die durch den Grundkörper 10 ausgefüllt ist. Das Versteifungselement 11 weist im distalen Bereich einen umlaufenden Ring 19 mit einem geschlossenen Querschnitt auf. Das Versteifungselement 11 dient zur Erhöhung der Eigenstabilität der Knöchelmanschette 1 und stellt gleichzeitig einen mechanischen Schutz gegen Umwelteinflüsse für das Knöchelgelenk oder die in dem Unterschenkelteil 4 angeordnete Elektronik bereit. Das Versteifungselement 11 kann auf dem Material des Grundkörpers 10 aufgeschweißt, aufgeklebt, aufgenäht oder aufgespritzt sein. Im dargestellten Ausführungsbeispiel ist das Versteifungselement 11 auf der Außenseite des Grundkörpers 10 angeordnet, es besteht auch die Möglichkeit, dass es auf der Innenseite des Grundkörpers angeordnet ist oder den Grundkörper 10 einschließt, also auf der Außen- und Innenseite des Grundkörpers 10 angeordnet ist.

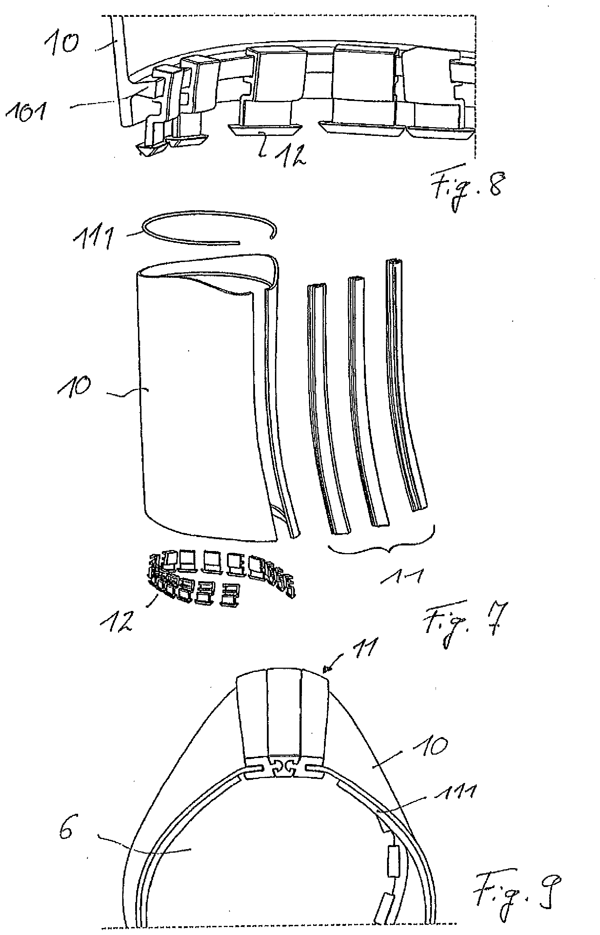

Eine nicht erfindungsgemäße Variante ist in der Figur 7 dargestellt, die eine Ausführungsform gemäß der Figur 1 eine Einzelteildarstellung zeigt. Der Grundkörper 10 ist als im Wesentlichen flächiger Zuschnitt ausgestaltet und so geformt, dass die einander gegenüberliegenden Kanten zusammengefügt werden können, um einen schlauchartigen Hohlkörper auszubilden. Das Versteifungselement 11 ist im dargestellten Ausführungsbeispiel mehrteilig ausgebildet und sieht zwei Seitenschienen vor, die an dem Grundkörper 10 festgelegt werden. Diese Seitenschienen werden über eine Mittelschiene formschlüssig miteinander verbunden. Die Mittelschiene weist dazu Ausnehmungen auf, in die Formschlusselemente der Seitenschienen eingreifen. Um eine Umfangsstabilität zu erreichen, ist ein weiteres Versteifungselement 111 vorgesehen, das im dargestellten Ausführungsbeispiel am proximalen Ende des Grundkörpers 10 angeordnet ist. Das Versteifungselement 111 kann als Federdraht oder Kunststoffspange ausgebildet sein.

An dem distalen Ende des Grundkörpers 10 sind separate Befestigungselemente 12 vorgesehen, die dort formschlüssig festgelegt sind und Vorsprünge aufweisen, mit denen die Befestigungselemente 12 und damit auch der Grundkörper 10 mit dem Versteifungselement 11 an dem Prothesenfuß festgelegt werden können.

Figur 8 zeigt eine vergrößerte Detailansicht des distalen Endes des Grundkörpers 10, an dem ein umlaufender Vorsprung 101 angeordnet ist, der beispielsweise daran angespritzt oder daran ausgebildet sein kann. Der Vorsprung 101 weist einen V-förmigen Querschnitt auf, der sich zur Außenwand des Grundkörpers 10 hin verjüngt. Die Befestigungselemente 12 weisen eine schwalbenschwanzartige Ausnehmung auf, so dass sie formschlüssig in den Vorsprung 101 eingreifen können. An dem distalen Ende der Befestigungselemente 12 sind pfeilartige Vorsprünge ausgebildet, um eine formschlüssige Verriegelung über das Ineingrifftreten mit korrespondierenden Ausnehmungen in dem Prothesenfuß zu ermöglichen. Die Befestigungselemente 12 sind reversibel und verschieblich auf dem Grundkörper 10 festgelegt, so dass unterschiedliche Anzahlen von Befestigungselementen an den jeweiligen Zuschnitten festgelegt werden können. Durch die Verschiebbarkeit der Befestigungselemente 12 an dem Grundkörper 10 ist es möglich, unterschiedliche Prothesenfußgrößen mit einem Zuschnitt des Grundkörpers 10 zu bedienen oder gegebenenfalls mit geringen Modifikationen eine Anpassung zu ermöglichen. Je größer die Prothesenfüße sind, desto größer ist der Abstand zwischen den einzelnen Ausnehmungen für die jeweiligen Befestigungselemente, so dass eine Anpassung ohne verschiebliche Befestigungselemente 12 nicht beliebig erfolgen kann.

Figur 9 zeigt die fertig gefügte Knöchelmanschette 1 mit dem Grundkörper 10, in dem in Umfangsrichtung wirkenden Versteifungselement 111 sowie dem in Längserstreckung wirkenden Versteifungselement 11 mit den zwei Außenschienen und der formschlüssig daran festgelegten Mittelschiene. Durch das Versteifungselement 11 wird ein geschlossener Hohlraum 6 ausgebildet, der die jeweilige Komponente der Protheseneinrichtung aufnimmt.

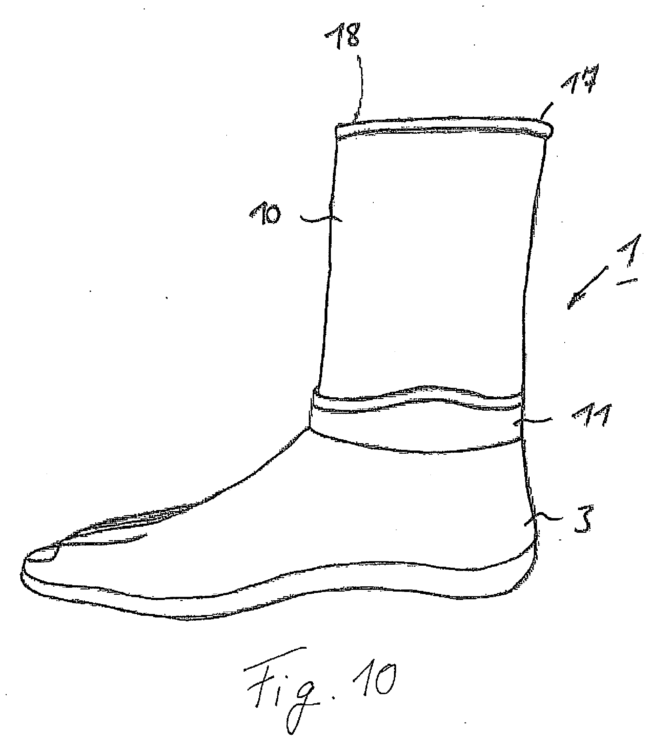

In der Figur 10 ist eine weitere Variante der Erfindung dargestellt. Die Variante entspricht im Wesentlichen der Ausführungsform gemäß der Figur 4, allerdings ist das Versteifungselement 11 im distalen Bereich des Grundkörpers 10 angeordnet, beispielsweise durch Aufspritzen, Anformen oder Ankleben einer separaten Materialschicht, um eine erhöhte Formstabilität im distalen Bereich der Knöchelmanschette 1 zu erreichen. Der proximale Abschnitt des Grundkörpers 10, also derjenige Abschnitt, der proximal zum Versteifungselement 11 angeordnet ist, weist eine ausreichende Eigenstabilität auf, um den Zwischenraum zwischen dem Prothesenfuß 3 und dem nicht dargestellten Unterschenkelteil 4 zu überbrücken.

## Patentansprüche

1. Knöchelmanschette für eine Protheseneinrichtung (2) mit einem Prothesenfuß (3) und einem Unterschenkelteil (4) zur Überbrückung eines zwischen dem Prothesenfuß (3) und dem Unterschenkelteil (4) vorhandenen Freiraums, mit einem Grundkörper (10) aus einem flexiblen Material, der einen Hohlraum (6) zur Aufnahme der Protheseneinrichtung (2) umfasst, wobei an dem Grundkörper (10) zumindest ein Versteifungselement (11) zur Erhöhung der Eigenstabilität der Knöchelmanschette (1) angeordnet ist, **dadurch gekennzeichnet, dass** das Versteifungselement (11) als Rahmen ausgebildet ist, der den Grundkörper (10) umgibt.

2. Knöchelmanschette nach Anspruch 1, **dadurch gekennzeichnet, dass** das Versteifungselement (11) aus einem Material mit einem gegenüber dem Material des Grundkörpers (10) größeren Verformungswiderstand besteht.

3. Knöchelmanschette nach Anspruch 1 oder 2, **dadurch gekennzeichnet, dass** der Grundkörper (10) aus einem Material hergestellt ist, das ein Textil und/oder ein Schaumstoff aufweist.

4. Knöchelmanschette nach einem der voranstehenden Ansprüche, **dadurch gekennzeichnet, dass** Befestigungselemente (12) zur formschlüssigen Festlegung der Knöchelmanschette (1) an dem Prothesenfuß (3) an dem Grundkörper (10) oder dem Versteifungselement (11) angeordnet sind.

5. Knöchelmanschette nach einem der voranstehenden Ansprüche, **dadurch gekennzeichnet, dass** in dem Versteifungselement (11) zumindest eine Scharnierenrichtung (13, 14) zur Erleichterung einer Flexion um ein Knöchelgelenk angeordnet ist.

6. Knöchelmanschette nach einem der voranstehenden Ansprüche, **dadurch gekennzeichnet, dass** die Knöchelmanschette (1) medial-lateral symmetrisch ausgebildet ist.

7. Knöchelmanschette nach einem der voranstehenden Ansprüche, **dadurch gekennzeichnet, dass** das Versteifungselement (11) den distalen Abschluss der Knöchelmanschette (1) bildet.

8. Knöchelmanschette nach einem der voranstehenden Ansprüche, **dadurch gekennzeichnet, dass** an der proximalen Innenseite des Grundkörpers (10) eine reibungsvermindernde Beschichtung (18) angeordnet ist.

9. Knöchelmanschette nach einem der voranstehenden Ansprüche, **dadurch gekennzeichnet, dass** das Versteifungselement (11) einen geschlossenen Querschnitt mit anterior und/oder posterior orientierten Ausschnitten (15, 16) aufweist.

10. Knöchelmanschette nach einem der Ansprüche 1 bis 8, **dadurch gekennzeichnet, dass** der Grundkörper (10) einen offenen Querschnitt aufweist und das Versteifungselement (11) den Querschnitt schließt.

## Claims

1. An ankle brace for a prosthetic device (2) comprising a prosthetic foot (3) and a below-knee part (4), said ankle brace being provided to bridge a free space between the prosthetic foot (3) and the below-knee part (4) and having a main body (10) which is made from a flexible material and which comprises a cavity (6) for receiving the prosthetic device (2), whereas the at least one reinforcement element (11) is arranged on the main body (10) in order to increase the inherent stability of the ankle brace (1), **characterized in that** the reinforcement element (11) is designed as a frame surrounding the main body (10).

2. The ankle brace as claimed in claim 1, **characterized in that** the reinforcement element (11) is made from a material that has a greater deformation resistance than the material of the main body (10).

3. The ankle brace as claimed in claim 1 or 2, **characterized in that** the main body (10) is produced from a material that has a textile and/or a foam.

4. The ankle brace as claimed in one of the preceding claims, **characterized in that** fastening elements (12) for securing the ankle brace (1) on the prosthetic foot (3) with form-fit engagement are arranged on the main body (10) or on the reinforcement element (11).

5. The ankle brace as claimed in one of the preceding claims, **characterized in that** at least one hinge mechanism (13, 14) for facilitating a flexion about an ankle joint is arranged in the reinforcement element (11).

6. The ankle brace as claimed in one of the preceding claims, **characterized in that** the ankle brace (1) is designed with medial-lateral symmetry.

7. The ankle brace as claimed in one of the preceding claims, **characterized in that** the reinforcement element (11) forms the distal end of the ankle brace (1).

8. The ankle brace as claimed in one of the preceding claims, **characterized in that** a friction-reducing coating (18) is arranged on the proximal inner face of the main body (10).

9. The ankle brace as claimed in one of the preceding claims, **characterized in that** the reinforcement element (11) has a closed cross section with cutouts (15, 16) oriented in the anterior and/or posterior direction.

10. The ankle brace as claimed in one of claims 1 through 8, **characterized in that** the main body (10) has an open cross section, and the reinforcement element (11) closes the cross section

## Revendications

1. Manchon de cheville pour un dispositif prothétique (2) comportant un pied prothétique (3) et une partie de bas de jambe (4) pour le pontage entre un espace libre présent entre le pied prothétique (3) et la partie de bas de jambe (4), comportant un corps de base (10) constitué en un matériau flexible et présentant une cavité (6) pour recevoir le dispositif prothétique (2), au moins un élément de rigidification (11) étant disposé sur le corps de base (10) pour augmenter la stabilité propre du manchon de cheville (1),
**caractérisé en ce que**
l'élément de rigidification (11) est réalisé sous forme de cadre qui entoure le corps de base (10).

2. Manchon de cheville selon la revendication 1,
**caractérisé en ce que**
l'élément de rigidification (11) est constitué en un matériau ayant une résistance à la déformation supérieure à celle du matériau du corps de base (10).

3. Manchon de cheville selon la revendication 1 ou 2,
**caractérisé en ce que**
le corps de base (10) est réalisé en un matériau qui comprend un textile et/ou une mousse.

4. Manchon de cheville selon l'une des revendications précédentes,
**caractérisé en ce que**
des éléments de fixation (12) pour l'immobilisation en coopération de forme du manchon de cheville (1) sur le pied prothétique (3) sont disposés sur le corps de base (10) ou sur l'élément de rigidification (11).

5. Manchon de cheville selon l'une des revendications précédentes,
**caractérisé en ce que**
au moins un moyen formant charnière (13, 14) pour faciliter une flexion autour d'une articulation de cheville est disposé dans l'élément de rigidification (11).

6. Manchon de cheville selon l'une des revendications précédentes,
**caractérisé en ce que**
le manchon de cheville (1) est réalisé à symétrie médiale-latérale.

7. Manchon de cheville selon l'une des revendications précédentes,
**caractérisé en ce que**
l'élément de rigidification (11) forme le raccord distal du manchon de cheville (1).

8. Manchon de cheville selon l'une des revendications précédentes,
**caractérisé en ce que**
un revêtement (18) réduisant la friction est disposé sur le côté intérieur proximal du corps de base (10).

9. Manchon de cheville selon l'une des revendications précédentes,
**caractérisé en ce que**
l'élément de rigidification (11) présente une section transversale fermée ayant des entailles (15, 16) d'orientation antérieure et/ou postérieure.

10. Manchon de cheville selon l'une des revendications 1 à 8,
**caractérisé en ce que**
le corps de base (10) présente une section transversale ouverte et l'élément de rigidification (11) referme la section transversale.
